# EUROPEAN PATENT APPLICATION

(11) **EP 3 505 508 A2**
(43) Date of publication of application: **03.07.2019**
(21) Application number: 17845584.6
(22) Date of filing: 29.08.2017
(51) Int. Cl.: C07C 53/122, C07C 53/124, A61K 31/185

(54) **COMPOSITION FOR USE IN DETECTING AN ALLERGY TO CLAVULANIC ACID**

(30) Priority: 29.08.2016 ES 201631133
(71) Applicant: Servicio Andaluz de Salud, 41071 Sevilla (ES); Universidad de Málaga, 29071 Málaga (ES)
(72) Inventor: MONTAÑEZ VEGA, María Isabel, 41071 Sevilla (ES); MAYORGA MAYORGA, Cristobalina, 41071 Sevilla (ES); TORRES JAEN, Maria José, 41071 Sevilla (ES); FERNÁNDEZ DUARTE, Tahía Diana, 41071 Sevilla (ES); ARIZA VEGUILLAS, Adriana, 41071 Sevilla (ES); SALAS CASINELLO, María, 41071 Sevilla (ES); PÉREZ INESTROSA VILLATORO, Ezequiel, 29071 Málaga (ES); NÁJERA ALBENDÍN, Francisco, 29071 Málaga (ES); BARBERO OLMOS, Nekane, 29071 Málaga (ES); VIDA POL, Yolanda, 29071 Málaga (ES)
(74) Representative: Hoffmann Eitle
(86) International application number: PCT/ES2017/070588
(87) International publication number: WO 2018/042066

(57) **Abstract**

The authors of the present invention describe compounds derived from or structures related to clavulanic acid and the medical uses thereof. The invention also relates to a composition, a dosage form, a kit or device, and the uses of same.

## Description

### FIELD OF THE INVENTION

The present invention is comprised in the field of medicine, pharmacy, and biological chemistry, and fundamentally relates to a composition of clavulanic acid derivatives at a given concentration, to the identification thereof, to the screening and/or diagnosis of an allergy to clavulanic acid (CLV), and to a diagnostic kit or device comprising said composition.

### BACKGROUND OF THE INVENTION

Beta-lactam (BL) antibiotics are the most common cause of drug allergies, with 10% of the general population being "labeled" as allergic to said antibiotics (Doña et al., 2014. J. Investig. Allergol. Clin. Immunol. 24:143-153). This has enormous implications since medical professionals must decide to choose an alternative antibiotic treatment which, though not the first choice, is more expensive and presents more side effects (Macy et al., 2014. Allergy Clin. Immunol. 133:790-796). The development of new antibiotics is not a priority for the pharmaceutical industry today, and since bacterial resistance rates are constantly increasing, BL allergy has become a global health issue (Solensky et al., 2014. J. Allergy Clin. Immunol. 133:797-798). However, less than 20% of adults and 10% of children considered allergic really are, therefore correct diagnosis is key to patient safety and health system sustainability (Doña et al., 2014. J. Investig. Allergol. Clin. Immunol. 24:143-153; Zambonino et al., 2014. Pediatr Allergy Immunol. 25:80-87).

All BLs can induce allergic reactions, with those mediated by IgE being the most common and most severe, and studied more in depth, and constituting the object of study of this project. Benzylpenicillin (BP) was the first BL involved, although since the end of the 1980s, it has been supplanted by amoxicillin (AX), which is the most relevant BL today (Torres et al., 2010. Allergy 56:850-856). In that sense, between 1999 and 2009 there was a relative increase in the frequency of skin tests with a positive reaction to AX of 79%, with a 38% drop in those testing positive to BP. However, this scenario continues to change around the world, since the combination of two BLs, amoxicillin-clavulanic acid (AX-CLV), is the combination most commonly involved in reactions, being from 10 to 35% between 2008 and 2010 (Doña et al., 2012. J. Investig. Allergol. Clin. Immunol. 22:363-371), and approaching 60% in 2014. This trend is confirmed in patients suspected of exhibiting an allergic reaction treated in the emergency rooms in three third-level hospitals in 2014, since the AX-CLV combination is responsible in 87.1% of the cases confirmed as allergic to BL (Barrionuevo et al., EAACI Meeting, 2015). This data reflects changes in consumer patterns, with a drop in use of AX (57-35.9%) and an increase in use of AX-CLV (39-61.8%) in Spain in the period between 1997 and 2009 (Lázaro *et al.,* 2010. Division de Farmacoepidemiologia y Farmacovigilancia (AEMPS)). These facts require a detailed re-assessment of the current BL allergy status.

Based on experimental models, for years it was considered that CLV was devoid of immunogenicity (Edwards et al., 1988. Int. Arch. Allergy Appl. Immunol. 85:184-189). However, in 2010 the authors published the first series of 77 patients with reactions to AX-CLV, identifying CLV as the agent responsible in 30% of the cases, clearly surpassing BP (6%); Torres et al., 2010. J. Allergy Clin. Immunol. 125:502-505 e502).

These results have been confirmed by other groups, which indicates that reactions to CLV today are close to 40% (Sanchez-Morillas et al., 2010. J. Allergy Clin. Immunol.126:177-179; Blanca-Lopez et al., 2010. Allergy 70:1013-1019). Furthermore, allergy to CLV cannot be modified despite the repeated administration of AX, since it does not induce cross-reactivity due to their structural differences (Blanca-Lopez et al., 2010. Allergy 70:1013-1019). This is important since an individual allergic to CLV after the administration of AX-CLV can safely use any other BL antibiotic, including AX, thereby augmenting the therapeutic arsenal, which does not occur in individuals allergic to AX (Torres et al., 2016. Clin. Exp. Allergy 46:264-274). Therefore, precise diagnosis of these patients is very important.

In recent years, the authors have been working on the design of tools for the diagnosis of an allergy to CLV, and on the incorporation thereof into clinical practice guidelines. The breakthroughs that have been made may include, among others, patent (P20093078) for the reagent (clavulanic acid, CLV) for skin tests being exploited by Diater laboratories, the basophil activation test or BAT (Torres et al., 2010. J. Allergy Clin. Immunol. 125:502-505 e502), and the histamine release test (HRT; Pineda et al., 2015. Intern. Arch. Allergy Immunol. 168:233-240), the latter being in collaboration with Diater laboratories. However, the diagnosis cannot be confirmed in up to 25% of patients who are allergic to AX-CLV since the sensitivity of skin tests and *in vitro* tests are not optimal. This means that a high percentage of patients with anaphylaxis will require for diagnosis a drug provocation test (DPT), which entails an increase in the cost and risk for the patient.

The complex chemical reactivity of CLV (Baggaley et al., 1997. Nat. Prod. Rep. 14:309-333; Martin et al., 1989. J. Chem. Soc., Perkin Transactions 2 223-226; Brethauer et al., 2008. J. Pharm. Sci. 97:3451-3455; Tremblay et al., 2008. Biochemistry 47:5312-5316) has hindered the identification of its antigenic determinants. After its conjugation to proteins, the resulting chemical structure is not stable, so very little is known about its immunogenicity (Torres et al., 2016. Clin.. Exp. Allergy 46:264-274). A nucleophilic attack by the protein against beta-lactam carbonyl of the CLV, with CLV-protein conjugates being generated, is assumed to be required in order to develop an allergic response to CLV. However, the instability of the resulting structure after conjugation leads to complex degradation pathways, giving rise to multiple possible determinants, which hinders identification of the molecules making up the antigenic determinant (Ariza et al., 2015. J. Investig. Allergol. Clin. Immunol. 25:12-25). Only one study, published in 1988, describes the intrinsic immunogenicity of CLV, which hypothesizes the formation of a low molecular weight determinant (such as oxopropanamide) together with other degradation products (Edwards et al., 1988. Int. Arch. Allergy Appl. Immunol. 85:184-189). Other more recent publications, not within the context of allergies, describe CLV bonding to beta-lactamases (Tremblay et al., 2008. Biochemistry 47:5312-5316; Rodkey et al., 2013. J. Am. Chem. Soc. 135:18358-18369) with proposed structures and conjugation mechanisms as described above. Though scarce in quantity, the available data points to the formation of low-density adducts of small and heterogeneous determinants, hindering immunogenicity (Lee et al., 1985. J. Immunol. Methods 84:235-243: Park et al., 1987. Biochem. Pharmacol. 36:581-590). Nevertheless, further studies are required to learn about the structure responsible for immediate hypersensitivity reactions to CLV and to therefore enable improving diagnostic tests.

Different synthetic approaches to potential determinants of the CLV, corresponding to different fragmentations of the drug after its binding to proteins for the immunological evaluation thereof in *in vitro* tests have been performed.

### BRIEF DESCRIPTION OF THE INVENTION

A **first aspect** of the invention relates to compounds structurally related to or derived from clavulanic acid, of general formula (I) or Clav, hereinafter compounds of the invention: 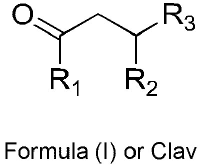 wherein
R1 are acid activating groups which are selected from halide, anhydrides, carbonate, or ester,
R2 is a group which is selected from aldehyde, alkoxy, or amino,
R3 is a group which is selected from hydrogen, alkoxy, or acid activating groups such as halides, anhydrides, or carbonates,
or any pharmaceutically acceptable salts, esters, tautomers, solvates, and hydrates thereof, or any combinations thereof.

In a preferred embodiment of this aspect of the invention, the compounds of the invention are selected from the list consisting of:
a) Clav 2, of formula (II): 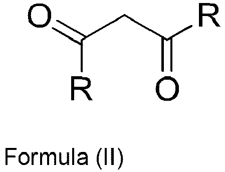 wherein
   R are acid activating groups which are selected from halide, anhydride, or carbonate,
   or any pharmaceutically acceptable salts, esters, tautomers, solvates, and hydrates thereof, or any combinations thereof,
b) Clav 5, of formula (III): 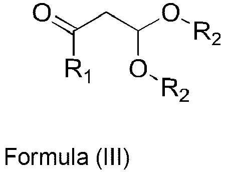 wherein
   R1 are acid activating groups which are selected from halide, anhydride, or carbonate,
   R2 is an alkyl group,
   or any pharmaceutically acceptable salts, esters, tautomers, solvates, and hydrates thereof, or any combinations thereof,
c) Clav 6, of formula (IV): 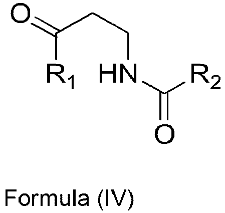 wherein
   R1 are acid activating groups which are selected from halide, anhydride, or carbonate,
   R2 is an alkyl group, or any pharmaceutically acceptable salts, esters, tautomers, solvates, and hydrates thereof, or any combinations thereof, and/or
d) Clav 7, of formula (V) (2-azetidinone): 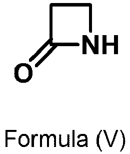 or any pharmaceutically acceptable salts, esters, tautomers, solvates, and hydrates thereof, or any combinations thereof.

In a preferred embodiment of this aspect of the invention, Clav 2 has formula (IIa): 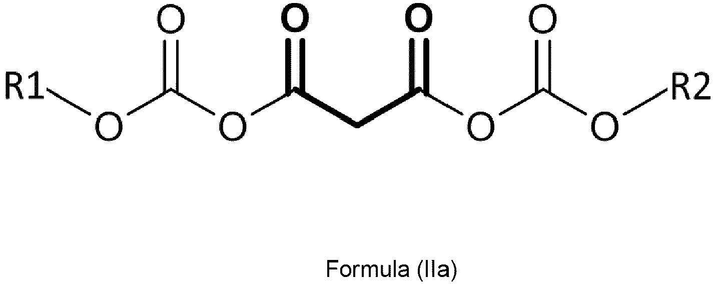 wherein R1 and R2 correspond to an alkyl group having 1 to 6 carbon atoms (C₁-C₆), such as 1 carbon atom, 2 carbon atoms, 3 carbon atoms, 4 carbon atoms, 5 carbon atoms, and/or 6 carbon atoms, substituted or unsubstituted, branched or unbranched, preferably 1 to 4 carbon atoms (C₁-C₄), more preferably 4 carbon atoms (C₄), substituted or unsubstituted, branched or unbranched, preferably unsubstituted and branched.

R1 and R2 can be the same or different. Preferably, R1 and R2 are the same.

Preferably, R1 and R2 are unsubstituted and branched alkyl groups having four carbon atoms, such as:

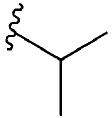

In another preferred embodiment of this aspect of the invention, Clav 2 is bis-((isobutoxycarbonyl)oxy)-1,3-dioxopropanoic anhydride, with formula (VI): 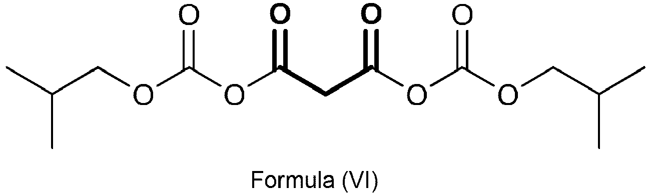 or any pharmaceutically acceptable salts, esters, tautomers, solvates, and hydrates thereof, or any combinations thereof.

In a preferred embodiment of this aspect of the invention, Clav 5 has formula (Ilia): 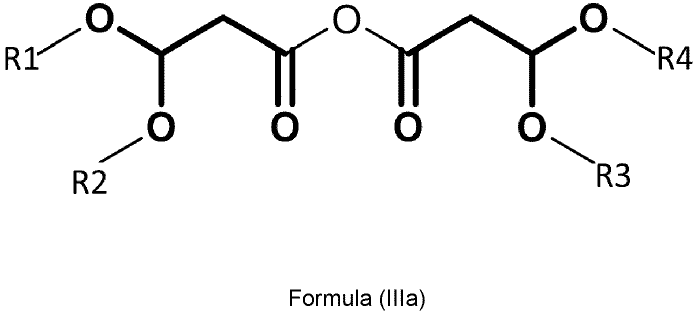 wherein R1, R2, R3, and R4, if they are present, correspond to an alkyl group having 1 to 6 carbon atoms (C₁-C₆), substituted or unsubstituted, branched or unbranched, preferably 1 to 4 carbon atoms (C₁-C₄), such as 1 carbon atom, 2 carbon atoms, 3 carbon atoms, and/or 4 carbon atoms, substituted or unsubstituted, branched or unbranched, preferably unsubstituted and unbranched, even more preferably 2 carbon atoms (C₂).

R1, R2, R3, and R4 can be the same or different. Preferably, R1, R2, R3, and R4 are present, are the same, and are an ethyl group,
or any pharmaceutically acceptable salts, esters, tautomers, solvates, and hydrates thereof, or any combinations thereof.

In another preferred embodiment of this aspect of the invention, Clav 5 is 3,3-diethoxypropanoic anhydride, with formula (VII): 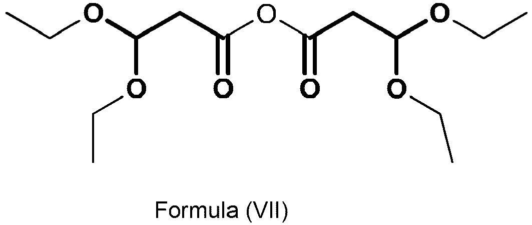 or any pharmaceutically acceptable salts, esters, tautomers, solvates, and hydrates thereof, or any combinations thereof.

In a preferred embodiment of this aspect of the invention, Clav 6 has formula IVa: 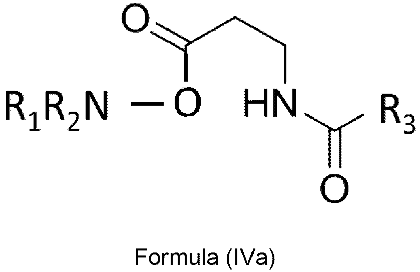 wherein R₁ corresponds to an H atom or a branched or unbranched, substituted or unsubstituted alkyl group having 1 to 6 carbon atoms (C₁-C₆), preferably a substituted or unsubstituted, branched or unbranched alkyl group having 2 to 5 carbon atoms (C₂-C₅), such as 2 carbon atoms, 3 carbon atoms, 4 carbon atoms, 5 carbon atoms, even more preferably a substituted and branched alkyl group having 4 carbon atoms (C₄);
wherein R₂ corresponds to a branched or unbranched, substituted or unsubstituted alkyl group having 1 to 6 carbon atoms (C₁-C₆), preferably a substituted or unsubstituted, branched or unbranched alkyl group having 2 to 5 carbon atoms (C₂-C₅), such as 2 carbon atoms, 3 carbon atoms, 4 carbon atoms, 5 carbon atoms, even more preferably a substituted and branched alkyl group having 4 carbon atoms (C₄);
or
wherein R₁ and R₂, together with the N atom to which they are bound, form a heterocyclic group with 4 to 7 carbon atoms, such as 4, 5, 6, or 7 carbon atoms, preferably 4 carbon atoms, optionally substituted with one or more oxo groups, such as with 1, 2, 3, or 4 oxo groups, preferably substituted with at least 2 oxo groups.

In a preferred embodiment, -NR₁R₂ corresponds to: 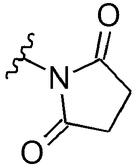 and wherein R₃ corresponds to an alkyl group having 1 to 6 carbon atoms (C₁-C₆), preferably a substituted or unsubstituted, branched or unbranched, preferably unsubstituted and unbranched alkyl group having 1 to 4 carbon atoms (C₁-C₄), such as 1 carbon atom, 2 carbon atoms, 3 carbon atoms, 4 carbon atoms, even more preferably an unsubstituted and unbranched alkyl group having 1 carbon atom (C₁) (a methyl group),
or any pharmaceutically acceptable salts, esters, tautomers, solvates, and hydrates thereof, or any combinations thereof.

In another embodiment, Clav6 has formula IVb: 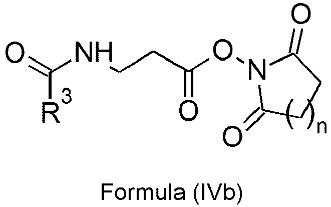 wherein n = 1, 2, or 3 and wherein R₃ corresponds to an alkyl group having 1 to 6 carbon atoms (C₁-C₆), preferably a substituted or unsubstituted, branched or unbranched, preferably unsubstituted and unbranched alkyl group having 1 to 4 carbon atoms (C₁-C₄), such as 1 carbon atom, 2 carbon atoms, 3 carbon atoms, 4 carbon atoms, even more preferably an unsubstituted and unbranched alkyl group having 1 carbon atom (C₁)(a methyl group),
or any pharmaceutically acceptable salts, esters, tautomers, solvates, and hydrates thereof, or any combinations thereof.

In another preferred embodiment of this aspect of the invention, Clav 6 is the ester 2,5-dioxopyrrolidin-1-yl 3-acetamidopropanoate, with formula (VIII): 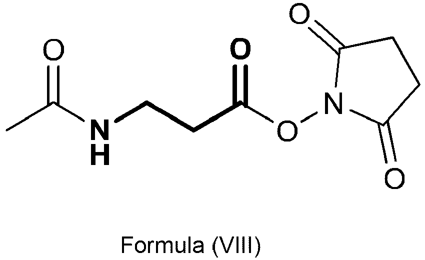 or any pharmaceutically acceptable salts, esters, tautomers, solvates, and hydrates thereof, or any combinations thereof.

Clav 7 is 2-azetidinone, with formula (V): 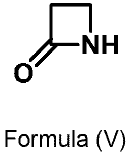 or any pharmaceutically acceptable salts, esters, tautomers, solvates, and hydrates thereof, or any combinations thereof.

A **second aspect** of the invention relates to a composition comprising or consisting of at least one compound of the invention, or any combinations thereof, hereinafter composition of the invention.

In a preferred embodiment of this aspect of the invention, the concentration of the compound of the invention is between 4 mM and 8 mM. More preferably, the concentration of the compound of the invention is 6 mM.

In another preferred embodiment, the composition of the invention comprises at least another active ingredient.

In another preferred embodiment, the composition of the invention comprises at least one pharmaceutically acceptable excipient.

In another preferred embodiment, the composition of the invention further comprises at least one pharmaceutically acceptable vehicle.

A **third aspect** of the invention relates to dosage form, hereinafter dosage form of the invention, comprising or consisting of at least one compound of the invention or the composition of the invention.

In a preferred embodiment of this aspect of the invention, the dosage form of the invention is selected from the list consisting of: plaster, ointment, paste, cream, solution, suspension, emulsion, lotion, liniment, gel, hydrogel, hydrocolloid, foam, powder, or any combinations thereof.

In a preferred embodiment of this aspect of the invention, the dosage form of the invention is a solution or suspension.

A **fourth aspect** of the invention relates to a kit or device comprising or consisting of at least one compound of the invention, the composition of the invention, or the dosage form of the invention.

In a preferred embodiment of this aspect of the invention, the kit or device of the invention further comprises the elements necessary for performing a basophil activation test (BAT).

In a preferred embodiment of this aspect of the invention, the kit or device of the invention further comprises at least one fluorescent monoclonal antibody suitable for the selection of basophils and the determination of the activation thereof, at least one stimulation buffer, at least one red blood cell lysis solution, at least one wash solution and human anti-lgE and N-formyl-methionyl-leucyl-phenylalanine and/or at least one positive control for the test.

A **fifth aspect** of the invention relates to the use of at least one compound of the invention, the composition of the invention, the dosage form of the invention, or the kit or device of the invention in the production of a medicinal product.

A **sixth aspect** of the invention relates to the use of at least one compound of the invention, the composition of the invention, the dosage form of the invention, or the kit or device of the invention in the production of a medicinal product for the prevention, identification, screening, and/or diagnosis of an allergy to clavulanic acid.

In a preferred embodiment of this aspect of the invention, the composition of the invention, the dosage form of the invention, or the kit or device of the invention are applied on an isolated biological sample. Preferably, the isolated biological sample is blood.

A **seventh aspect** of the invention relates to an *in vitro* method of obtaining data for use for the prevention, identification, screening, and/or diagnosis of an allergy to clavulanic acid in an individual, hereinafter first method of the invention, which method comprises:
a) quantifying the percentage of activated basophils in an isolated biological sample of said individual at the baseline, and
a') quantifying the percentage of activated basophils in an isolated biological sample of said individual after adding to same a compound of the invention, the composition of the invention, the dosage form of the invention, or applying the kit or device of the invention.

In a preferred embodiment of this aspect of the invention, the quantification of the percentage of activated basophils is performed by means of the markers expressed on the basophil cell surface. In another preferred embodiment of the first method of the invention, the basophil cell surface activation marker is selected from the list consisting of CD203c, CD63, CD13, CD107a, CD164, CD80, CD86, CD40L, HLA-DR, CD123, CRTh2, Ph-Creb, Ph-STAT5, Ph-S6rp, Ph-eIIF4E, and CREB. Preferably, the activation marker is CD63.

In another preferred embodiment of this aspect of the invention, the quantification of the percentage of cells expressing the activation marker is performed by means of flow cytometry.

In a preferred embodiment of this aspect of the invention, the first method of the invention further comprises:
b) calculating a stimulation index.
   In a preferred embodiment of this aspect of the invention, the "stimulation index" is the percentage of activated basophils according to step a) with respect to the percentage of activated basophils at the baseline according to step a').
   In another preferred embodiment of this aspect of the invention, the isolated biological sample of step a) is blood. In another preferred embodiment of this aspect of the invention, additionally, the blood is previously incubated with a stimulation buffer and/or is centrifuged.
   In a preferred embodiment of this aspect of the invention, the quantification of markers is performed by means of flow cytometry.
   An **eighth aspect** of the invention relates to a method for the prevention, identification, screening, and/or diagnosis of an allergy to clavulanic acid, hereinafter second method of the invention, which method comprises steps (a)-(b) of the first method of the invention, which method further comprises:
c) assigning the individual of step a) to the group of individuals with an allergy to clavulanic acid when the stimulation index is equal to or greater than 2, more preferably greater than 2.5, and even more preferably greater than 3.

### DESCRIPTION OF THE DRAWINGS

**Figure 1****.** Proposed clavulanic acid determinants corresponding to the resulting fragments after conjugation to proteins (antigenic determinant I and antigenic determinant II) and equivalent structures (Clav 1-Clav 7) for evaluation in BAT.
**Figure 2****.** Specific structures used in BAT corresponding to Clav 1- Clav 7.
**Figure 3****.** Results of the BAT using CLV structures, Clav 1, Clav 2, Clav 3, and Clav 4, obtained in 25 patients selective for CLV and 15 tolerant controls, expressed as stimulation index (SI). Statistical analysis using the Mann-Whitney test, significant differences (p<0.0001) indicated in the graph.
**Figure 4****.** Analysis of the percentages of positivity in the BAT results obtained in 25 patients selective for CLV (black bars) and 15 tolerant controls (white bars).
**Figure 5****.** Results of the BAT using the CLV, and the activated structures Clav 2 and Clav 5. Top: 7 patients selective for CLV with a high response to Clav 2 and 6 tolerant controls. Bottom: 3 patients selective for CLV and 6 tolerant controls. Statistical analysis using the Mann-Whitney test, significant differences * (p<0.05) and ** (p<0.01) indicated in the graph.
**Figure 6****.** Analysis of the percentages of positivity in the BAT results obtained in 7 patients selective for CLV (black bars) and 6 tolerant controls (white bars).
**Figure 7****.** Results of the inhibition of basophil activation with wortmannin (WTM) after stimulation with CLV, Clav 2, and Clav 5. The bars show the stimulation index (SI) obtained for basophil activation not inhibited and inhibited with WTM. The significant differences were analyzed with the Wilcoxon test and are indicated with * (p<0.05) and ** (p<0.01).

### DETAILED DESCRIPTION OF THE INVENTION

The authors of the present invention have obtained a series of results which show that a specific series of structures related to or derived from clavulanic acid (Clav 2, Clav 5, Clav 6, and/or Clav 7) could be use in the prevention, screening, identification, and/or diagnosis of individuals with an allergy to clavulanic acid (CLV).

### COMPOUNDS OF THE INVENTION

Therefore, a **first aspect** of the invention relates to compounds structurally related to or derived from clavulanic acid, of general formula (I) or Clav, hereinafter compounds of the invention: 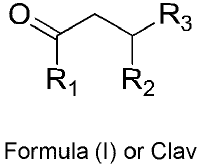 wherein
R1 are acid activating groups which are selected from halide, anhydrides, carbonate, or ester.
R2 is a group which is selected from aldehyde, alkoxy, or amino.
R3 is a group which is selected from hydrogen, alkoxy, or acid activating groups such as halides, anhydrides, or carbonates.
or any pharmaceutically acceptable salts, esters, tautomers, solvates, and hydrates thereof, or any combinations thereof.

Preferably, the compound of general formula (I) or Clav according to the first aspect of the present invention is *N*-butyl-3,3-dimethoxypropanamide, as indicated in the following formula (Formula la), or any pharmaceutically acceptable salts, esters, tautomers, solvates, and hydrates thereof, or any combinations thereof:

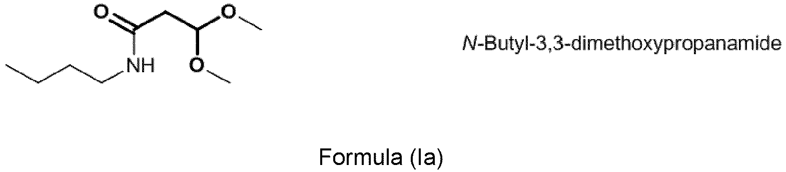

In a preferred embodiment of this aspect of the invention, the compounds of the invention are selected from the list consisting of:
a) Clav 2, of formula (II): 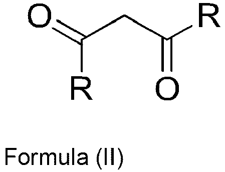 wherein
   R are acid activating groups which are selected from halide, anhydride, or carbonate,
   or any pharmaceutically acceptable salts, esters, tautomers, solvates, and hydrates thereof, or any combinations thereof,
b) Clav 5, of formula (III): 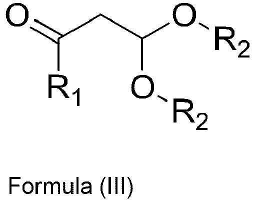 wherein
   R1 are acid activating groups which are selected from halide, anhydride, or carbonate,
   R2 is an alkyl group,
   or any pharmaceutically acceptable salts, esters, tautomers, solvates, and hydrates thereof, or any combinations thereof,
c) Clav 6, of formula (IV): 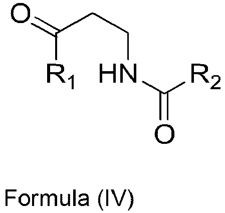 where
   R1 are acid activating groups which are selected from halide, anhydride, or carbonate,
   R2 is an alkyl group,
   or any pharmaceutically acceptable salts, esters, tautomers, solvates, and hydrates thereof, or any combinations thereof, and/or
d) Clav 7, of formula (V) (2-azetidinone): 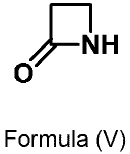 or any pharmaceutically acceptable salts, esters, tautomers, solvates, and hydrates thereof, or any combinations thereof.

In a preferred embodiment, only one compound of the invention is used. In another preferred embodiment, any combination of the compounds of the invention is used.

In a preferred embodiment of this aspect of the invention, Clav 2 has formula (IIa): 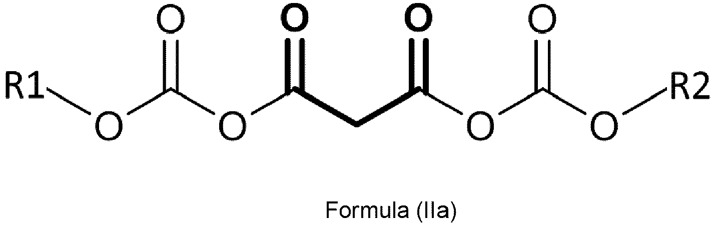 wherein R1 and R2 correspond to an alkyl group having 1 to 6 carbon atoms (C₁-C₆), such as 1 carbon atom, 2 carbon atoms, 3 carbon atoms, 4 carbon atoms, 5 carbon atoms, and/or 6 carbon atoms, substituted or unsubstituted, branched or unbranched, preferably 1 to 4 carbon atoms (C₁-C₄), more preferably 4 carbon atoms (C₄), substituted or unsubstituted, branched or unbranched, preferably unsubstituted and branched.

R1 and R2 can be the same or different. Preferably, R1 and R2 are the same.

Preferably, R1 and R2 are unsubstituted and branched alkyl groups having four carbon atoms, such as:

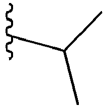

In another preferred embodiment of this aspect of the invention, Clav 2 is bis-((isobutoxycarbonyl)oxy)-1,3-dioxopropanoic anhydride, with formula (VI): 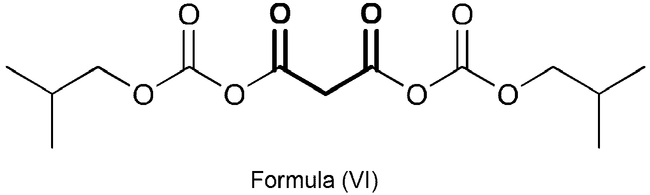 or any pharmaceutically acceptable salts, esters, tautomers, solvates, and hydrates thereof, or any combinations thereof.

In another preferred embodiment of this aspect of the invention, Clav 3 is 3-amino-N-butylpropanamide, of formula (IX), or any pharmaceutically acceptable salts, esters, tautomers, solvates, and hydrates thereof, or any combinations thereof:

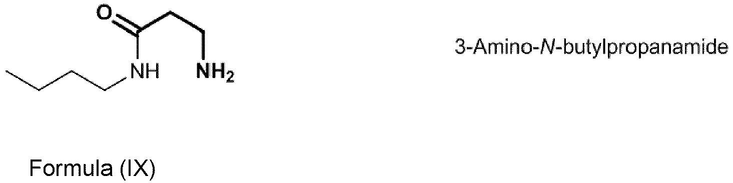

In another preferred embodiment of this aspect of the invention, Clav 4 is 3-aminopropanoic acid, of formula (X), or any pharmaceutically acceptable salts, esters, tautomers, solvates, and hydrates thereof, or any combinations thereof:

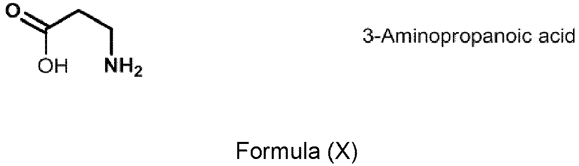

In a preferred embodiment of this aspect of the invention, Clav 5 has formula (Ilia): 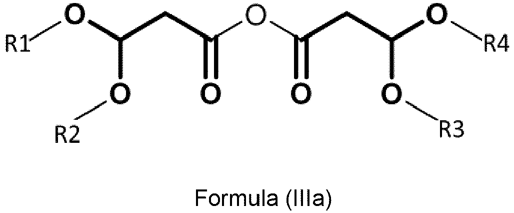 wherein R1, R2, R3, and R4, if they are present, correspond to an alkyl group having 1 to 6 carbon atoms (C₁-C₆), substituted or unsubstituted, branched or unbranched, preferably 1 to 4 carbon atoms (C₁-C₄), such as 1 carbon atom, 2 carbon atoms, 3 carbon atoms, and/or 4 carbon atoms, substituted or unsubstituted, branched or unbranched, preferably unsubstituted and unbranched, even more preferably 2 carbon atoms (C₂).

R1, R2, R3, and R4 can be the same or different. Preferably, R1, R2, R3, and R4 are present, are the same, and are an ethyl group (C₂),
or any pharmaceutically acceptable salts, esters, tautomers, solvates, and hydrates thereof, or any combinations thereof.

In another preferred embodiment of this aspect of the invention, Clav 5 is 3,3-diethoxypropanoic anhydride, with formula (VII): 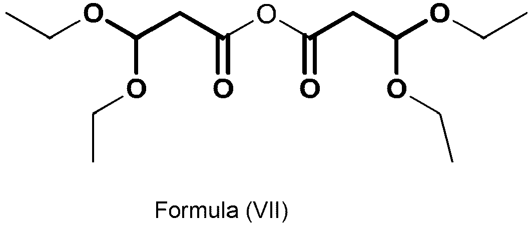 or any pharmaceutically acceptable salts, esters, tautomers, solvates, and hydrates thereof, or any combinations thereof.

In another preferred embodiment of this aspect of the invention, Clav 5 is 3,3-dimethoxypropanoic (isobutylcarbonic) anhydride (Clav 5-bis, of formula (XI)), or any pharmaceutically acceptable salts, esters, tautomers, solvates, and hydrates thereof, or any combinations thereof:

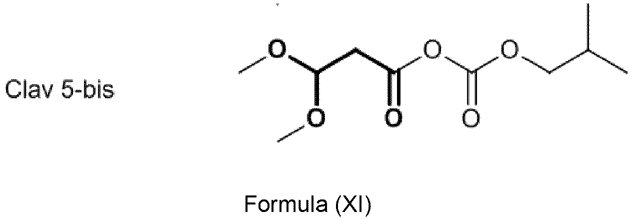

In a preferred embodiment of this aspect of the invention, Clav 6 has formula (IVa): 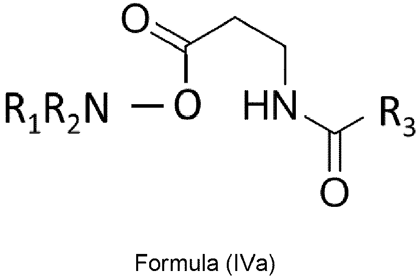 wherein R₁ corresponds to an H atom or a branched or unbranched, substituted or unsubstituted alkyl group having 1 to 6 carbon atoms (C₁-C₆), preferably a substituted or unsubstituted, branched or unbranched alkyl group having 2 to 5 carbon atoms (C₂-C₅), such as 2 carbon atoms, 3 carbon atoms, 4 carbon atoms, 5 carbon atoms, even more preferably a substituted and branched cyclic alkyl group having 4 carbon atoms (C₄);
wherein R₂ corresponds to a branched or unbranched, substituted or unsubstituted alkyl group having 1 to 6 carbon atoms (C₁-C₆), preferably a substituted or unsubstituted, branched or unbranched alkyl group having 2 to 5 carbon atoms (C₂-C₅), such as 2 carbon atoms, 3 carbon atoms, 4 carbon atoms, 5 carbon atoms, even more preferably a substituted and branched cyclic alkyl group having 4 carbon atoms (C₄);
or
wherein R₁ and R₂, together with the N atom to which they are bound, form a heterocyclic group with 4 to 7 carbon atoms, such as 4, 5, 6, or 7 carbon atoms, preferably 4 carbon atoms, optionally substituted with one or more oxo groups, such as with 1, 2, 3, or 4 oxo groups, preferably substituted with at least 2 oxo groups.

In a preferred embodiment, -NR₁R₂ corresponds to: 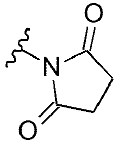 and wherein R₃ corresponds to an alkyl group having 1 to 6 carbon atoms (C₁-C₆), preferably a substituted or unsubstituted, branched or unbranched, preferably unsubstituted and unbranched alkyl group having 1 to 4 carbon atoms (C₁-C₄), such as 1 carbon atom, 2 carbon atoms, 3 carbon atoms, 4 carbon atoms, even more preferably an unsubstituted and unbranched alkyl group having 1 carbon atom (C₁)(a methyl group),
or any pharmaceutically acceptable salts, esters, tautomers, solvates, and hydrates thereof, or any combinations thereof.

In another embodiment, Clav6 has formula (IVb): 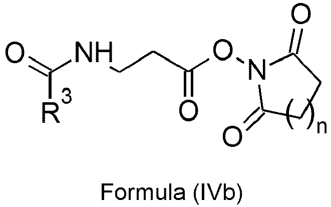 wherein n = 1, 2, or 3 and wherein R³ corresponds to an alkyl group having 1 to 6 carbon atoms (C₁-C₆), preferably a substituted or unsubstituted, branched or unbranched, preferably unsubstituted and unbranched alkyl group having 1 to 4 carbon atoms (C₁-C₄), such as 1 carbon atom, 2 carbon atoms, 3 carbon atoms, 4 carbon atoms, even more preferably an unsubstituted and unbranched alkyl group having 1 carbon atom (C₁)(a methyl group),
or any pharmaceutically acceptable salts, esters, tautomers, solvates, and hydrates thereof, or any combinations thereof.

In another preferred embodiment of this aspect of the invention, Clav 6 is the ester 2,5-dioxopyrrolidin-1-yl 3-acetamidopropanoate, with formula (VIII): 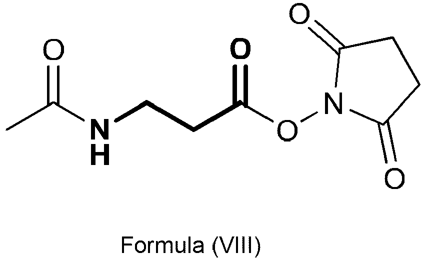 or any pharmaceutically acceptable salts, esters, tautomers, solvates, and hydrates thereof, or any combinations thereof.

Clav 7 is 2-azetidinone, with formula (V): 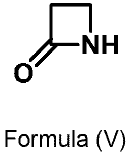 or any pharmaceutically acceptable salts, esters, tautomers, solvates, and hydrates thereof, or any combinations thereof.

On the other hand, "clavulanic acid" understood herein as (3*R*,5*R*,*Z*)-2-(2-hydroxyethylidene)-7-oxo-1-oxa-4-aza-bicyclo[3.2.0]heptane-3-carboxylic acid, of formula (XII): 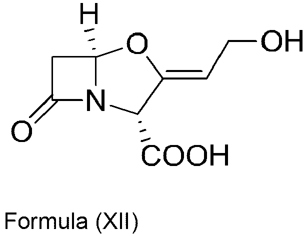 with CAS number 58001-44-8, is a beta-lactamase enzyme inhibitor which is used against resistance to the beta-lactam antibiotics the bacteria secreting this enzyme exhibit. It is most commonly found in the form of potassium clavulanate salt.

### COMPOSITION OF THE INVENTION

A **second aspect** of the invention relates to a composition comprising or consisting of at least one compound of the invention, or any combinations thereof, hereinafter composition of the invention.

In a preferred embodiment of this aspect of the invention, the concentration of the compound of the invention is between 4 mM and 8 mM, more preferably between 5 mM and 7 mM, and even more preferably 6 mM.

In another preferred embodiment, the composition of the invention comprises at least another active ingredient. Preferably, the composition of the invention further comprises clavulanic acid, and even more preferably clavulanic acid in the form of potassium salt.

In another preferred embodiment, the composition of the invention comprises at least one pharmaceutically acceptable excipient.

In another preferred embodiment, the composition of the invention further comprises at least one pharmaceutically acceptable vehicle.

In another preferred embodiment, the composition of the invention is a pharmaceutical composition.

Likewise, the prodrugs of the pharmaceutical compositions of the invention are within the scope of the present invention. As it is used herein, the term "prodrug" includes any derivative of a compound of the invention, such as a compound of formula (I), as a non-limiting example: esters (including carboxylic acid esters, amino acid esters, phosphate esters, sulfonate esters of metallic salts, etc.), carbamates, amides, etc., which, upon administration to an individual or upon addition to an isolated biological sample of an individual, can be transformed directly or indirectly into said compound, such as the compound of formula (I), in the mentioned individual. Advantageously, said derivative is a compound that increases the bioavailability of the compound when administered to an individual or added to an isolated biological sample of an individual, or enhances the release of the compound in a biological compartment. The preparation of said prodrug can be carried out by means of conventional methods known to those skilled in the art.

The "derivatives", including both pharmaceutically acceptable compounds, i.e., derivatives of the compound of formula (I) that may be used in the production of a medicinal product or food compositions, and pharmaceutically unacceptable derivatives, since these may be useful in the preparation of pharmaceutically acceptable derivatives, are within the scope of the present invention. The derivatives may include stereoisomers, depending on the presence of multiple bonds or chiral centers. The stereoisomers (enantiomers, diastereoisomers, meso compounds) and mixtures thereof fall within the scope of the present invention, i.e., the term stereoisomer also refers to any mixture of stereoisomers (racemic stereoisomers or mixtures of the different stereoisomers in different proportions). The diastereoisomers, as well as the mixtures thereof, can be separated by means of conventional techniques.

Any of the compounds of the invention can be in crystalline form, or in liquid state, for example oils, as free compounds or as solvates. In this sense, as it is used herein, the term "solvate" includes both pharmaceutically acceptable solvates, i.e., solvates of the compound that may be used in the production of a medicinal product, and pharmaceutically unacceptable solvates, which can be used in the preparation of pharmaceutically acceptable solvates or salts. The nature of the pharmaceutically acceptable solvate is not critical, provided that it is pharmaceutically acceptable. In a particular embodiment, the solvate is a hydrate. The solvates can be obtained by conventional solvation methods known to those skilled in the art.

For their application in diagnosis or therapy as pharmaceutical compositions, the compounds of the invention, the salts, prodrugs, or solvates thereof, will preferably be in a pharmaceutically acceptable or substantially pure form, i.e., they will present a pharmaceutically acceptable level of purity excluding the usual pharmaceutical additives such as diluents and carriers, and not including material considered toxic at normal dosage levels. The levels of purity for the active ingredient are preferably greater than 50%, more preferably greater than 70%, and still more preferably greater than 90%. In a preferred embodiment, they are greater than 95% of the compound of formula (I) or the salts, solvates, or prodrugs thereof.

Moreover, it should be mentioned that the pharmaceutically acceptable vehicles and adjuvants which may be used in the pharmaceutical compositions of the invention are the adjuvants and vehicles known to those skilled in the art and commonly used in the production of therapeutic compositions.

The compounds described in the present invention, the salts, prodrugs, and/or solvates thereof, as well as the pharmaceutical compositions containing them may be used together with other additional drugs or active ingredients to provide a combination therapy. Said additional drugs may be part of the same pharmaceutical composition or, alternatively, may be provided in the form of a separate composition for administration at the same time or at a different time with respect to the administration of the pharmaceutical composition comprising a compound of formula (I) or a salt, prodrug, or solvate thereof.

In another preferred embodiment of this aspect of the invention, the medicinal product for the prevention of allergy is a vaccine.

As it is used herein, the term "medicinal product" or "pharmaceutical composition" refers to any substance used for the diagnosis of diseases in humans and animals. In the context of the present invention, the medicinal product is used for the diagnosis of a disease, and preferably the disease is an allergic disease, more preferably an allergy to clavulanic acid.

As used herein, the terms "active ingredient", "active substance", "pharmaceutically active substance", or "pharmaceutically active ingredient" mean any component which potentially provides a different activity in the basophil or another different effect in the diagnosis, or affects the structure or function of the body of humans or other animals. The term includes those components which promote a chemical change in the production of the drug and are present therein in an envisaged modified form which provides the specific activity or effect.

The composition or pharmaceutical composition of the present invention can be formulated for administration to an animal, and more preferably to a mammal, including humans, or in an isolated biological sample thereof in a range of forms known in the state of the art. In that sense, they may be, without limitation, in a sterile aqueous solution or in biological fluids, such as serum. The aqueous solutions may or may not be buffered and have additional active or inactive components. The additional components include salts to modulate ionic strength and/or preservatives including, but without limitation to, antimicrobial agents, antioxidants, chelating agents, and the like. Alternatively, the compositions can be prepared for administration or use in solid form. The compositions can be combined with several inert vehicles or excipients, including, but without limitation to: binders such as microcrystalline cellulose, tragacanth gum, or gelatin; excipients such as starch or lactose; dispersing agents such as alginic acid or corn starch; lubricants such as magnesium stearate; glidants such as colloidal silicon dioxide; sweetening agents such as sucrose or saccharin, or flavoring agents such as mint or methyl salicylate. Preferably, the composition of the invention will be added to an isolated biological sample, preferably blood or a product obtained therefrom coming from a mammal, preferably from a human.

The concentration for obtaining a diagnostically effective amount will depend on the compound in question. In the sense used in this description, the expression "diagnostically effective amount" refers to the amount of compound that produces the desired effect and will generally be determined, among other causes, by the characteristics typical of said compounds. The "adjuvants" and "pharmaceutically acceptable vehicles" which may be used in said compositions are the vehicles known to those skilled in the art.

The term "prevention" as it is understood in the present invention consists of preventing the onset of the disease, i.e., preventing the disease or pathological condition from occurring in a subject (preferably a mammal, and more preferably a human), particularly where said subject has a predisposition for the pathological condition. In this case, the pathological condition is an allergic reaction to clavulanic acid

### DOSAGE FORM OF THE INVENTION

A **third aspect** of the invention relates to dosage form, hereinafter dosage form of the invention, comprising or consisting of at least one compound of the invention or the composition of the invention.

In a preferred embodiment of this aspect of the invention, the dosage form of the invention is selected from the list consisting of: plaster, ointment, paste, cream, solution, suspension, emulsion, lotion, liniment, gel, hydrogel, hydrocolloid, foam, powder, or any combinations thereof.

In a preferred embodiment of this aspect of the invention, the dosage form of the invention is a solution or suspension.

"Dosage form" is understood herein as the mixture of one or more active ingredients with or without additives having physical characteristics for their suitable

The compounds and compositions of the present invention may be used together with other medicinal products or compounds in combined diagnostic therapies or techniques. The other drugs may be part of the same composition or of another different composition, for use in diagnosis at the same time or at different times.

### KIT OR DEVICE OF THE INVENTION

A **fourth aspect** of the invention relates to a kit or device comprising or consisting of at least one compound of the invention, the composition of the invention, or the dosage form of the invention.

In a preferred embodiment of this aspect of the invention, the kit or device of the invention further comprises the elements necessary for performing a basophil activation test, for example, although without limitation, at least one fluorescent monoclonal antibody suitable for the selection of basophils and the determination of the activation thereof, at least one stimulation buffer, at least one red blood cell lysis solution, at least one wash solution and human anti-IgE and N-formyl-methionyl-leucyl-phenylalanine and/or at least one positive control for the test.

The kit or device of the invention may include another additional device for taking the isolated biological sample (preferably blood).

The "basophil activation test" or "BAT" is an *in vitro* functional test which detects the activation of basophils after a stimulus with a specific antigen by detecting the expression of cell surface activation markers, which is usually monitored by flow cytometry using fluorescent monoclonal antibodies. Therefore, this test is used for the *in vitro* diagnosis of allergies. The technique comprises several steps: drawing blood and preparing the solution or suspension, performing stimulation with allergen(s), performing cellular marking with fluorescent antibodies which discriminate activated basophils, and performing counting in a flow cytometer. The technique can be carried out using as the substrate whole blood or the buffy coat previously isolated from the whole blood sample.

### MEDICAL USES OF THE INVENTION

A **fifth aspect** of the invention relates to the use of at least one compound of the invention, the composition of the invention, the dosage form of the invention, or the kit or device of the invention in the production of a medicinal product, or alternatively, to the compound(s) of the invention, the composition of the invention, the dosage form of the invention, or the kit or device of the invention for use in medicine.

A **sixth aspect** of the invention relates to the use of at least one compound of the invention, the composition of the invention, the dosage form of the invention, or the kit or device of the invention in the production of a medicinal product for the prevention, identification, screening, and/or diagnosis of an allergy to clavulanic acid, or alternatively, to the compound(s) of the invention, the composition of the invention, the dosage form of the invention, or the kit or device of the invention for use in the prevention, identification, and/or diagnosis of the allergy to clavulanic acid.

In a preferred embodiment of this aspect of the invention, the composition of the invention, the dosage form of the invention, or the kit or device of the invention are applied on an isolated biological sample. Preferably, the isolated biological sample is blood.

Additionally, at least one compound of the invention, the composition of the invention, the dosage form of the invention, or the kit or device of the invention may be used in other *in vitro* assays, such as, for example, though without limitation, in immunoassays, histamine release test, lymphocyte proliferation assay, ELISpot), and *in vivo* assays (skin test) for the diagnosis of allergy to CLV.

### METHODS OF THE INVENTION

A **seventh aspect** of the invention relates to an *in vitro* method of obtaining data for use for the prevention, identification, screening, and/or diagnosis of an allergy to clavulanic acid in an individual, hereinafter first method of the invention, which method comprises:
a) quantifying the percentage of activated basophils in an isolated biological sample of said individual at the baseline, and
a') quantifying the percentage of activated basophils in an isolated biological sample of said individual after adding to same a compound of the invention, the composition of the invention, the dosage form of the invention, or applying the kit or device of the invention.

In a preferred embodiment of this aspect of the invention, the quantification of the percentage of activated basophils is performed by means of the markers expressed on the basophil cell surface. In another preferred embodiment of the first method of the invention, the basophil cell surface activation marker is selected from the list consisting of CD203c, CD63, CD13, CD107a, CD164, CD80, CD86, CD40L, HLA-DR, CD123, CRTh2, Ph-Creb, Ph-STAT5, Ph-S6rp, Ph-eIIF4E, and CREB. Preferably, the activation marker is CD63.

In another preferred embodiment of this aspect of the invention, the quantification of the percentage of cells expressing the activation marker is performed by means of flow cytometry. In a preferred embodiment of this aspect of the invention, the first method of the invention further comprises:
b) calculating a stimulation index.
   In a preferred embodiment of this aspect of the invention, the "stimulation index" is the percentage of activated basophils according to step a) with respect to the percentage of activated basophils at the baseline according to step a').
   In another preferred embodiment of this aspect of the invention, the isolated biological sample of step a) is blood. In another preferred embodiment of this aspect of the invention, additionally, the blood is previously incubated with a stimulation buffer and/or is centrifuged.
   In a preferred embodiment of this aspect of the invention, the quantification of markers is performed by means of flow cytometry.
   An **eighth aspect** of the invention relates to a method for the prevention, identification, screening, and/or diagnosis of an allergy to clavulanic acid, hereinafter second method of the invention, which method comprises steps (a)-(b) of the first method of the invention, which method further comprises:
c) assigning the individual of step a) to the group of individuals with an allergy to clavulanic acid when the stimulation index is equal to or greater than 2, more preferably greater than 2.5, and even more preferably greater than 3.

Alternatively, the first method of the invention comprises the following steps:
a) quantifying the amount of IgE antibody in an isolated biological sample of said individual after adding to same a compound of the invention, the composition of the invention, the dosage form of the invention, or applying the kit or device of the invention.
b) comparing the amount obtained in step a) with a negative control sample, where the negative control sample is a sample from the same individual of the step with nothing added to it.
c) assigning the individual of step a) to the group of individuals with an allergy to clavulanic acid, when the value obtained in step a) is greater than the negative control described in step b).

As it is used in the description, the term "comparison" refers but is not limited to the comparison of the result of the amplification of the biological sample to be analyzed, also called the biological test sample, with amplification of one or several reference samples being desirable. The reference sample can be analyzed, for example, simultaneously or consecutively, together with the biological test sample. The comparisons described in the methods of the present invention can be performed manually or in a computer-aided manner.

As used herein, an "individual" or "subject" refers to a human or non-human mammal under observation, and more preferably a human being. The individual may be either an individual predisposed to allergy to clavulanic acid or an individual who suffers from the allergy.

Steps (a)-(a') and/or (b) of the method described above may be completely or partially automated, for example, by means of sensory robotics for the quantification in steps (a)-(a') or the comparison in step (b).

Another **aspect** of the invention relates to a computer program comprising programming instructions to make a computer carry out to practice the process according to any of the methods of the invention.

In particular, the invention comprises computer programs arranged on or in a carrier. The carrier may be any entity or device capable of supporting the program. When the program is incorporated in a signal which can be transported directly by a cable or another device or means, the carrier may be made up of said cable or another device or means. As a variant, the carrier may be an integrated circuit in which the program is included, with the circuit integrated being adapted for executing, or for being used in the execution of, the corresponding processes.

For example, the programs may be incorporated in a storage medium, such as a ROM memory, a CD ROM memory, or a semiconductor ROM memory, a USB memory, or a magnetic storage medium, for example, a floppy disk or a hard drive. Alternatively, the programs may be stored in a transmittable carrier signal. For example, it may be an electrical or optical signal that can be transported by the electrical or optical cable, by radio, or by any other means.

The invention also extends to computer programs adapted so that any processing means may carry out to practice the methods of the invention. Such programs may be in the form of source code, object code, an intermediate source code and object code, for example, in partially compiled form, or in any other form suitable for use in putting into practice the processes according to the invention. The computer programs also comprise cloud applications based on said method.

Therefore, another **aspect** of the invention relates to a computer-readable storage medium comprising programming instructions capable of making a computer carry out the steps of any of the methods of the invention.

Another **aspect** of the invention relates to a transmittable signal comprising programming instructions capable of making a computer carry out the steps of any of the methods of the invention.

Throughout the description and claims, the word "comprises" and variants thereof do not seek to exclude other technical features, additions, components, or steps. For those skilled in the art, other objects, advantages, and features of the invention will be inferred in part from the description and in part from the practice of the invention. The following examples and drawings are provided by way of illustration and do not seek to limit the present invention.

### EXAMPLE OF THE INVENTION

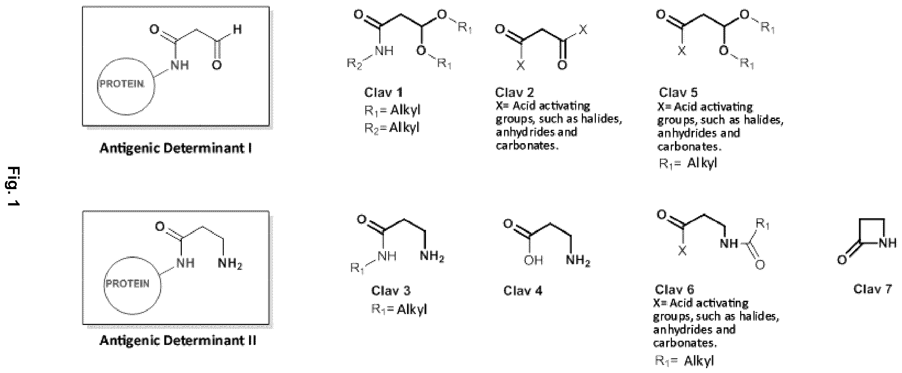

Method which allows the evaluation of an immediate allergic reaction to clavulanic acid with suitable sensitivity. The use of structures related to clavulanic acid (with formulas Clav 2, Clav 5, and Clav 6 described in Figure 1) in the basophil activation test (BAT) significantly raises sensitivity of the technique compared with the use of the clavulanic acid molecule *per se.*

### Patients

The study included 30 patients with an immediate hypersensitivity reaction to CLV after taking AX-CLV. The diagnosis was confirmed by means of skin test or drug provocation test (DPT) for penicillin, AX, or CLV following the European Network for Drug Allergy (ENDA) guidelines. Twenty-five cases with confirmed, good tolerance to AX-CLV and a negative skin test for major and minor determinants of benzylpenicillin (PA), amoxicillin (AX), and clavulanic acid (CLV) were selected as a control group.

The patient samples were provided by the Biobank of Hospital Regional of Malaga (Biobank of the Andalusian Public Health System), immediately frozen after reception, and processed following current procedures. The study was performed according to the principles of the Declaration of Helsinki and was approved by the Provincial Ethics Committee of Malaga. All the subjects included in the study were orally informed and signed the corresponding informed consent.

### Skin tests

The maximum concentrations used were the following: 0.04 mg/ml benzylpenicilloyl octa-L-lysine (PPL), 0.5 mg/ml minor determinant (MD), 20 mg/ml AX, and 20 mg/ml CLV (all provided by DIATER Laboratories, Madrid, Spain). In those cases where the subject has a history of severe anaphylaxis, the test was performed with increasing dilutions until reaching the maximum concentration, as commonly described in the literature. In the skin tests, a papule larger than 3 mm with a negative response to the saline control was considered positive. In the intradermal tests, the zone of the papule was marked at the beginning and 20 minutes after the test. An increase in diameter larger than 3 mm was considered positive.

### Drug Provocation Test (DPT)

To confirm the selective reactions, a placebo-controlled simple, blind DPT was performed, using BP and AX at increasing doses under strict hospital supervision (as described in Torres et al. Allergy 2003; 58:961-72). In summary, for subjects with negative skin test for PPL and DM, the DPT was performed with BP by parenteral route with increasing doses until reaching the cumulative dose (10⁶ IU), followed by a 2-day course of PV therapy at 500 mg/8h. If the DPT with BP and VP was negative and the skin test was negative for AX, a DPT was performed with AX using incremental doses with 30-minute time intervals until reaching a cumulative dose of 500 mg, and this was followed by a 2-day course of therapy with AX at 500 mg/8h.

### In vitro determination of specific immunoglobulin E antibodies

This was performed by means of the CAP-FEIA system (Pharmacia diagnostics, Uppsala, Sweden), using c1 (benzylpenicilloyl, BPO) and c6 (amoxicilloyl, AXO), and following the manufacturer's instructions; the results were obtained by direct comparison with standards run in parallel, and considering values greater than 0.35 kUA/L positive.

### Preparation of synthetic determinants of clavulanic acid

**N-butyl-3,3-dimethoxypropanamide (Clav1):** In a Schlenk flask (oven-dried and cooled under nitrogen), butylamine (3.5 ml, 35 mmol) and commercial methyl 3,3-dimethoxypropanoate (0.7 ml, 5 mmol) were added, being kept under stirring at 60°C for four days. The reaction mixture was cooled, diluted with dichloromethane (10 ml), and washed with 10 ml of 2M HCI. The organic fraction was dried on anhydrous MgSO₄, filtered, and concentrated under vacuum, the pure target compound being obtained as a yellow oil (1.05 g). ¹H NMR (400 MHz, CDCl₃): δ 0.89 (3H, t, *J* = 7.3 Hz, CH₃), 1.27-1.36 (2H, m, CH₂), 1.43-1.51 (2H, m, CH₂), 2.55 (2H, d, *J* = 5.3 Hz, CH₂CH), 3.17-3.26 (2H, m, CH₂NH), 3.35 (6H, s, 2 x OMe), 4.67 (1H, t, *J* = 5.3 Hz, CHCH₂), 6.08 (1H, bs, NH); ¹³C NMR (100 MHz, CDCl₃): δ 13.8 (CH3), 20.1 (CH₂), 31.6 (CH₂), 39.2 (CH₂), 41.0 (CH2), 54.2 (2 x OCH₃), 102.3 (CH), 169.1 (CO). HRMS (ESI) m/z (C₉H₁₉NO₃ + Na, 212.1263), found: 212.1258.

**3-amino-*N-*butylpropanamide (Clav3):** Previously prepared *N*-butyl-3-(1,3-dioxoisoindolin-2-yl)propanamide (147 mg, 0.53 mmol) and hydrazine hydrate (4 ml) were heated at 100°C for three hours in EtOH (5 ml). The solution was subsequently extracted with CH₂Cl₂ (3 x 10 ml), dried on anhydrous MgSO₄, filtered, and concentrated under low pressure to provide the pure target compound in the form of a white solid (50 mg, 65%).¹H NMR (400 MHz, MeOD): δ 0.94 (3H, t, J = 7.3 Hz, CH₃), 1.33-1.40 (2H, m), 1.45-1.52 (2H, m), 2.39 (2H, t, *J* = 6.6 Hz), 2.94 (2H, t, *J* = 6.6 Hz), 3.18 (2H, t, *J* = 7.0 Hz); ¹³C NMR (100 MHz, MeOD): δ 14.1 (CH₃), 21.1 (CH₂), 32.5 (CH₂), 37.9 (CH₂), 38.6 (CH₂), 40.0 (CH₂), 173.7 (CO). HRMS (ESI) *m*/*z* (C₇H₁₆N₂O+H, 145.1335), found: 145.1335.

**3-amino-propanoic acid (Clav4):** This compound was commercially acquired from Sigma Aldrich (Saint Louis, USA).

**3,3-diethoxypropanoic anhydride (Clav5):** 3,3-diethoxypropanoic acid (300 mg, 1.85 mmol) was dissolved under nitrogen atmosphere in dry CH₂Cl₂ (5 ml) and cooled at 0°C. EDCI (177 mg, 0.92 mmol) was slowly added and the reaction mixture was left under stirring overnight, slowly reaching room temperature. The reaction mixture was then washed with water (2 x 5 ml). The organic fraction was dried on anhydrous MgSO₄, filtered, and concentrated under low pressure for obtaining the pure target compound in the form of a brownish oil (198 mg, 71%). ¹H NMR (400 MHz, CDCl₃): δ 1.23 (12H, t, *J* = 7.0 Hz, 4 x CH₃), 2.82 (4H, d, *J* =5.8 Hz, 2 x CH₂CO), 3.59 (4H, m, 2 x CH₂), 3.70 (4H, m, 2 x CH₂), 4.97 (2H, t, *J* = 5.8 Hz, 2 x CH); 13 C NMR (100 MHz, CDCl₃): δ 15.3 (4 x CH3), 41.0 (2 x CH2CO), 62.5 (4 x CH₂CH₃), 99.0 (2 x CH), 165.3 (2 x CO); HRMS (ESI) m/z (C₁₄H₂₆O₇ + Na, 329.1576), found: 329.1571.

**2,5-Dioxopyrrolidin-1-yl 3-acetamidopropanoate (Clav6):** 3-acetamidopropanoic acid (201 mg, 1.5 mmol) was dissolved under nitrogen atmosphere in dry CH₂Cl₂ (5 ml) and cooled at 0°C. EDCI (352 mg, 1.8 mmol) and N-hydroxysuccinimide (211 mg, 1.8 mmol) were slowly added, and the reaction mixture was left to stir, reaching room temperature overnight. The solution obtained was washed with water (2 x 5 ml), the organic fraction was dried on anhydrous MgSO₄, filtered, and concentrated under vacuum for obtaining the pure target compound in the form of a colorless oil (175 mg, 50%). ¹H NMR (400 MHz, CDCl₃): δ 1.99 (3H, s, CH₃), 2.81-2.88 (6H, m, 3 x CH₂), 3.63-3.68 (2H, m, CH₂NH), 6.26 (1H, bs, NH). ¹³C NMR (100 MHz, CDCl₃): δ 22.8 (CH₃), 25.5 (2 x CH₂), 31.4 (CH₂), 34.8 (CH₂), 167.3 (CO), 169.3 (2 x CO), 170.7 (CO). HRMS (ESI) m/z (C₉H₁₂N₂O₅+H, 229.0824), found: 229.0812.

### Basophil activation by flow cytometry

The BAT was performed as described by Torres MJ *et al.* (Torres MJ, et al. Clin Exp Allergy 2004; 34:1768-75) with CLV (Sigma Aldrich, Saint Louis, USA). The different synthesized structures were assayed at different concentrations: 8, 4, 2, and 1 mM. The cells were stained with monoclonal antibodies, anti-CD63-FITC, CD203c-PE, CCR3-APC (Caltag Laboratories, Burlingame, CA), and measured in a FACSCalibur flow cytometer (Becton-Dickinson Bioscience, San Jose, CA) acquiring at least 500-1000 basophils per sample. The results were analyzed with FlowJo® software (Tree Star, Inc. USA), and the activation was expressed as a stimulation index (SI), calculated as the ratio between the percentage of activated basophils (CD63 + or CD203c + cells) in samples stimulated with different haptenes and non-stimulated sample. To calculate the SI, the percentage of spontaneously activated basophils was required to be equal to or greater than 5%, as described by Sanz ML *et al.* (Sanz ML, et al. Clin Exp Allergy 2002; 32:277-86).

To confirm that the activation of basophils was mediated by IgE, the inhibitory effect of wortmannin at 1 µM was analyzed. Wortmannin acts as a specific inhibitor of phosphatidylinositol 3-kinase (PI3-K), which has proven to be one of the most important FceRI receptor-activated kinases involved in human basophil IgE-mediated stimulation. This was used with the positive control (anti-IgE) and the different haptenes and synthesized structures at the same concentrations described above.

### In vitro studies of HSA (human serum albumin) modification with clavulanic acid

The HSA concentration-dependent modification by CLV was investigated *in vitro.* Human serum albumin (10 mg/ml, 0.15 mM) was incubated in PBS with a recently prepared solution of CLV in PBS at increasing concentrations, at 37°C for 16 h. The molar ratios of HSA:CLV used for incubation were: 1:0, 1:10, 1:20, 1:40, 1:80, 1:160, 1:320, and 1:600.

The HSA-CLV conjugates were filtered through dialysis filters and washed several times with bidistilled water. The protein fraction was analyzed by means of MALDI-TOF MS, whereas the low molecular weight wash fractions were lyophilized and analyzed by ¹H-RMN. The analysis of the low molecular weight wash fractions analyzed by ¹H-RMN showed the presence of small amounts of CLV after incubation when the lowest concentrations of CLV were used; however, in fractions derived from a 1:600 molar ratio considerable amounts of CLV were found after incubation, in addition to other colored degradation products. MALDI-TOF results of the protein fractions indicated saturation of HSA-bound CLV when the 1:320 molar ratio was used, which is consistent with the excess CLV found after incubation for the conditions which used higher concentrations of CLV.

HSA modified with the minimum concentration of CLV was analyzed by means of liquid chromatography-mass spectrometry (Orbitrap).

***Analysis by mass spectrometry-liquid chromatography (Orbitrap):*** The samples of each of the digestates, control and HSA samples treated with clavulanic acid, treated with Arg-C, trypsin, and chymotrypsin, were analyzed using an LTQ Velos-Orbitrap mass spectrometer (Thermo Fisher Scientific, Bremen, Germany) coupled to a nano-HPLC system (Proxeon, Denmark) and an Impact HD high-resolution Q-TOF spectrometer (Bruker, Bremen) coupled to a nano-HPLC (Proxeon, Denmark). The peptide mixtures were initially concentrated in a Proxeon nanotrap column, 100 µm ID, 2 cm packing with Reprosil C18, particle size 5 mm (Proxeon, Denmark), and were then separated using a analytical reversed-phase nano column.

***Orbitrap analysis:*** An EASY column, 75 mm in diameter, 10 cm in length and packed with Reprosil, particle size 3 µm, was used as the analytical column (Proxeon, Denmark). An acetonitrile gradient (5-35% ACN/formic acid at 0.1% in water, in 22 min, flow rate of 300 nL/min) was used to elute the peptides through a stainless steel nanospray emitter (Proxeon, Denmark) over the nanospray ionization source of the LTQ Velos-Orbitrap mass spectrometer. MS/MS fragmentation (200 ms, 100-2800 m/z) of twenty of the most intense ions was carried out when their intensity exceeded a minimum threshold of 1000 counts, as detected from a 500 ms MS scanner (300-1500 m/z), using a dynamic exclusion time of 20 seconds for selecting precursors and excluding individually charged ions.

***Q-TOF impact analysis:*** An Acclaim PepMap 15 cm column (Dionex) 75 µm was used for separation. Chromatography was performed using a 0.1% formic acid-acetonitrile gradient (5-35% in 22 min, flow rate 300 nL/min). The column was coupled to the mass spectrometer input through a Captive Spray ionization source (Bruker). The MS acquisition was adjusted to MS cycles (1 Hz), followed by MS/MS (0.5-2 Hz) of the 8 most intense precursor ions with a fragmentation intensity threshold of 5000 counts, and using a dynamic exclusion time of 0.5 min and the exclusion of individually charged ions. All the spectra were acquired in the 100-2200 Da interval. LC-MSMS data was analyzed using Data Analysis 4.2 software (Bruker).

***Identification of peptides:*** Proteins were identified using Mascot (Matrix Science, London UK) to search against the human serum albumin isoform 1 sequence; ALBU_HUMAN. The MS/MS spectra were sought with a mass tolerance of the precursor of 10 ppm, tolerance of the fragment of 0.7 Da (Orbitrap) or 0.05 Da (Impacto), and the corresponding enzyme specificity. Cysteine carbamidomethylation was established as the fixed modification, and methionine oxidation and clavulanic acid modifications were established as variable modifications. The specificity for modifications of the clavulanic acid fragment was defined in Cys, Hys, and Lys as well as in the N-terminal protein.

### Molecular modeling

The crystalline structures of HSA and clavulanic acid, considered in their carboxylate form (CLV), were obtained from RCSB Protein Data Bank (PDB ID: 1AO6 and PDB ID: J01) (Sugio et al., 1999. Protein Eng 12:439-46). The relaxation of A chain of HSA was performed with AMBER 12 MD software package and parm99 force field, using a complete atomistic simulation. To preserve charge neutrality, a suitable number of chloride counterions was added. Then the protein was solvated into a truncated octahedral cell, with a minimum solvation layer of 10 \ ring {A} around the structure and the TIP3P 12 water model. To retain the experimental folding, the solvated structure was minimized, keeping the backbone atoms fixed during the initial cyclo phase of minimization of the conjugated gradient. The optimized structure obtained was used in docking analysis.

For each recognized addition site, a docking search was performed using AutoDock 4.2 13 software and a 14 Å sphere around the lysine adducts (in their neutral form) was considered. CLV was docked in this grid with the Lamarckian algorithm and the flexible ligands were left free to rotate. The genetic algorithm generated 100 poses and the other parameters were left at the default settings. The obtained complexes were classified considering both the docking scores and the distance between the lysine amino group and the CLV β-lactam carbonyl group.

### Statistical analysis

Comparisons of the quantitative variables without a normal distribution were performed using Mann-Whitney and Kruskall-Wallis tests. The comparisons of the qualitative variables were performed by means of the X² test. Evolution of the MTDs was analyzed by means of survival analysis (Log-rank Mantel-Cox statistical test). All indicated P values represent two-tailed tests, with values <0.05 being considered statistically significant.

### RESULTS

Molecules derived from CLV which correspond to the different proposed determinants have been synthesized: antigenic determinant I (aldehyde derivative: oxopropanamide type; Clav 1, Clav 2, and Clav 5) and antigenic determinant II (amine derivative: β-alanine type; Clav 3, Clav 4, Clav 6, and Clav 7). See Figure 1.

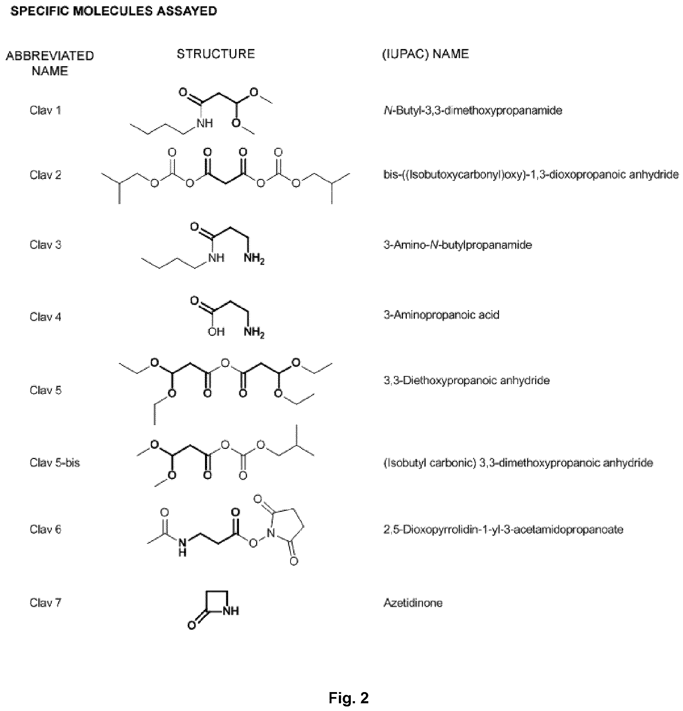

Structures this type together with CLV "per se" have been evaluated in the BAT in subjects selective for CLV. See specific structures used in Figure 2.

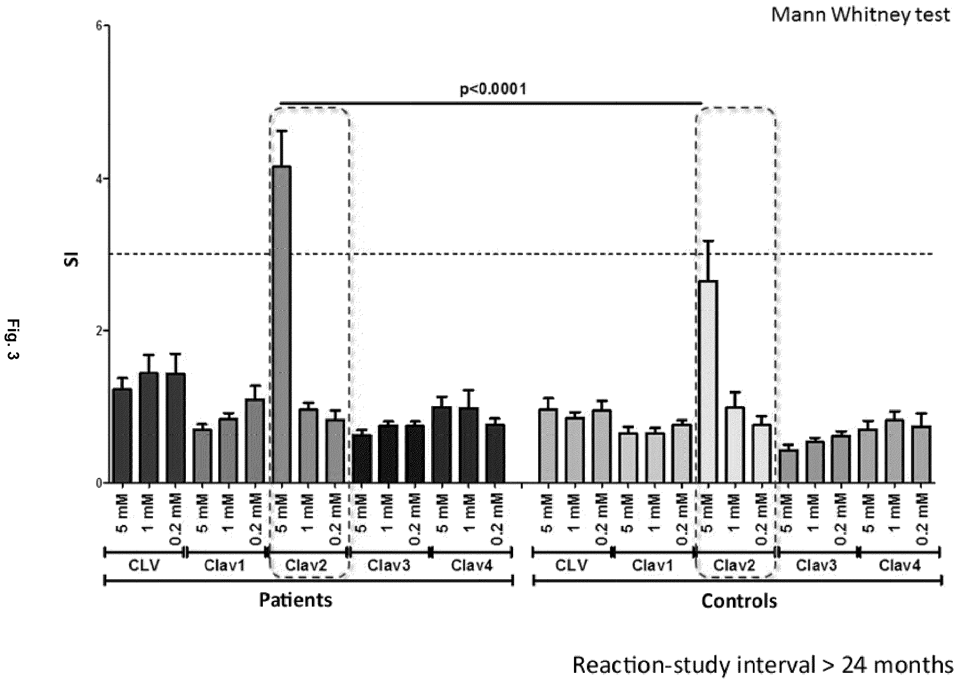

In a first phase, structures Clav 1, Clav 2, Clav 3, and Clav 4 were tested at different concentrations (5, 1, and 0.2 mM) in 25 patients selective for CLV and in 15 tolerant controls. The results are shown in Figure 3, where it can be seen that only Clav 2 provided positive results (at the highest concentration), even in those patients with negative BAT for CLV. The stimulation index with Clav 2 was significantly higher in patients compared to the tolerant controls (p<0.0001).

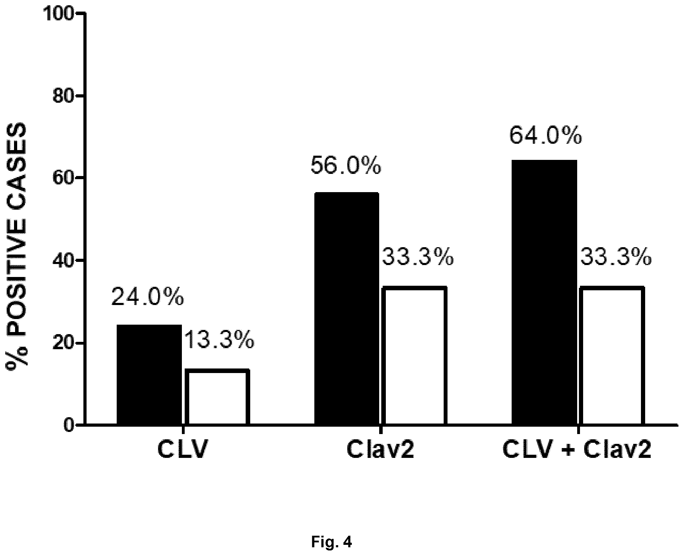

The percentage of positive cases, depicted in Figure 4, was analyzed using a cutoff point for CLV > 1.5 and for Clav 2 > 3, according to the results obtained in the ROC curves. It was observed that in patients selective for CLV, the percentage of positive cases went up from 24% with CLV to 56% with Clav 2 and to 64% if both results were combined. In the case of tolerant subjects, the percentage of positive cases was 13.3% with CLV, 33.3% with Clav2, and 33.3% with the combination of both.

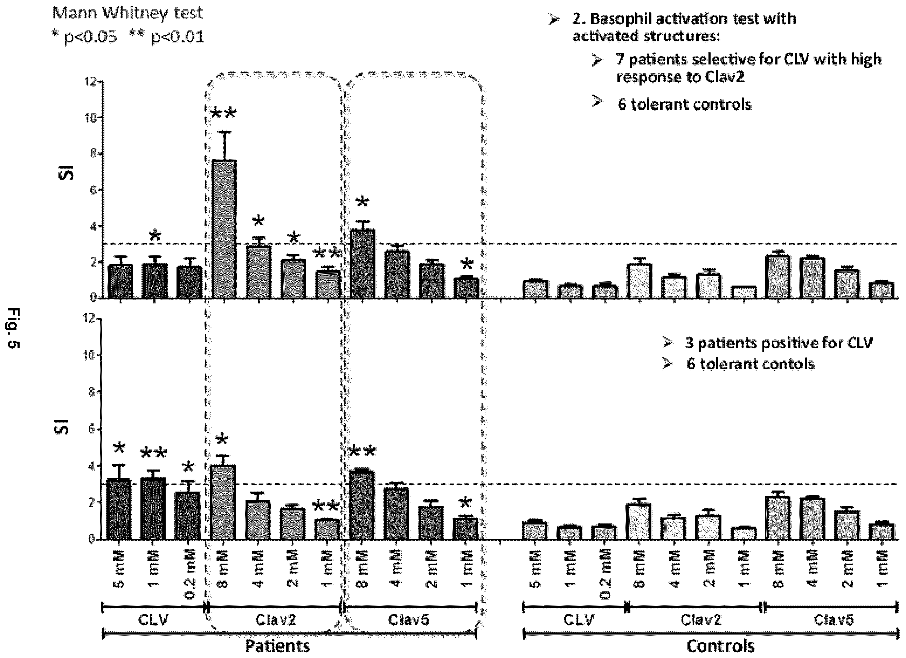

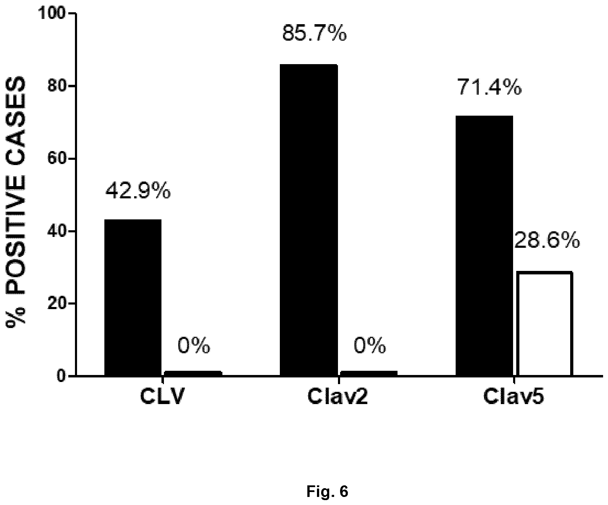

BAT was subsequently performed with a new structure, Clav 5, which, like Clav 2, exhibited a higher protein binding capacity. Clav 2 and Clav 5 were tested at a higher range of concentrations (8, 4, 2, and 1 mM) in 7 patients positive for Clav 2 in the preceding test and in 6 controls, obtaining higher stimulation indices for both structures in patients compared to controls at the highest concentrations (p=0.003 for Clav 2 and p=0.015 for Clav 5 at 8 mM; p=0.015 for Clav 2 at 4 mM), results shown in Figure 5. Using the same cutoff point as in the preceding test, the percentage of positivity was analyzed as shown in Figure 6. The percentage of positive cases is 85.7% for Clav 2 and 71.4% for Clav 5. In this case, there was no positive case for Clav 2 among the tolerant controls and at 28.6% for Clav 5.

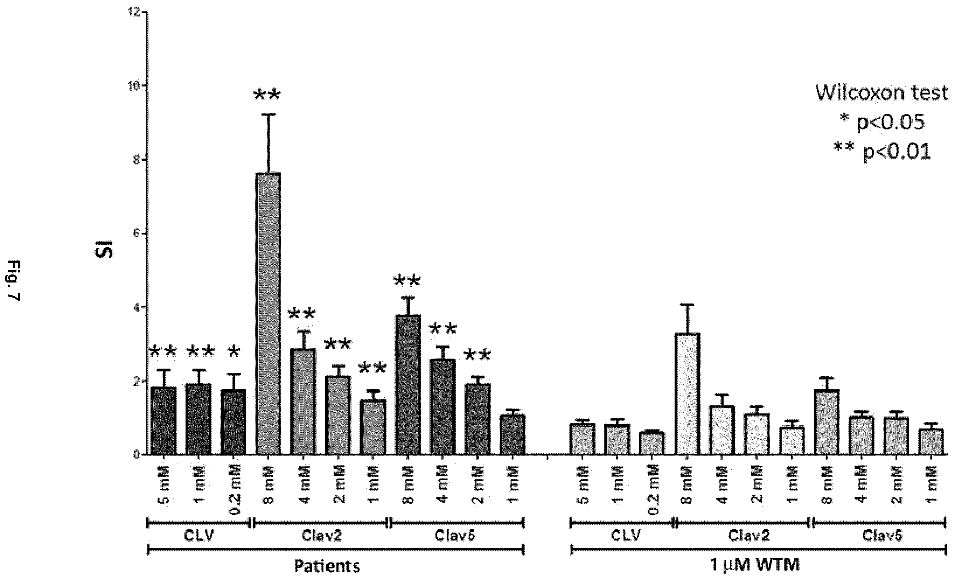

Finally, an inhibition assay to inhibit the IgE-mediated basophil activation pathway, using WTM, was performed with both structures (Clav 2 and Clav 5). The results of the BAT including prior incubation with WTM at 1 µM (p=0.002 for Clav2 and p=0.001 for Clav 5 at 8 mM; p=0.001 for Clav 2 and p=0.001 for Clav 5 at 4 mM) proved that basophil activation is IgE-mediated and specific (Figure 7).

The structures derived from antigenic determinant I which can bind to proteins (Clav 2 and Clav 5) activate the basophils in patients selective for CLV via IgE. These molecules significantly increase BAT sensitivity for the diagnosis of patients with immediate reactions to CLV.

## Claims

1. Compounds structurally related to or derived from clavulanic acid, of general formula (I) or Clav: 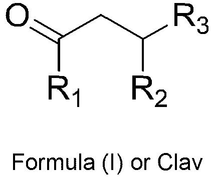 wherein
R1 are acid activating groups which are selected from halides, anhydrides or carbonates or ester,
R2 is a group which is selected from aldehyde, alkoxy, or amino,
R3 is a group which is selected from hydrogen, alkoxy, or acid activating groups such as halides, anhydrides or carbonates,
or any pharmaceutically acceptable salts, esters, tautomers, solvates, and hydrates thereof, or any combinations thereof,
wherein said compounds structurally related to or derived from clavulanic acid are selected from the group consisting of:
a) Clav 2, of formula (IIa): 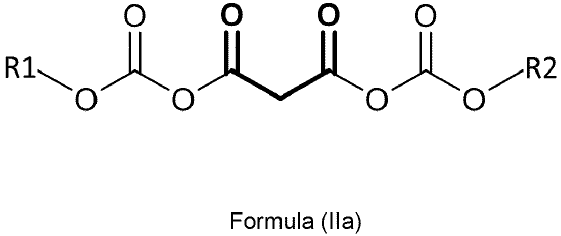 wherein R1 and R2 correspond to a substituted or unsubstituted, branched or unbranched alkyl group having 1 to 6 carbon atoms (C₁-C₆),
or any pharmaceutically acceptable salts, esters, tautomers, solvates, and hydrates thereof, or any combinations thereof;
b) Clav 5, of formula (IIIa): 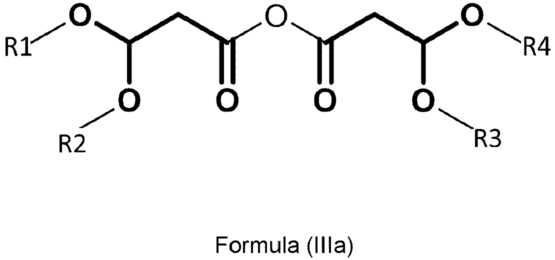 wherein R1, R2, R3, and R4, if they are present, correspond to a substituted or unsubstituted, branched or unbranched alkyl group having 1 to 6 carbon atoms (C₁-C₆),
or any pharmaceutically acceptable salts, esters, tautomers, solvates, and hydrates thereof, or any combinations thereof;
c) Clav 6, of formula (IVa): 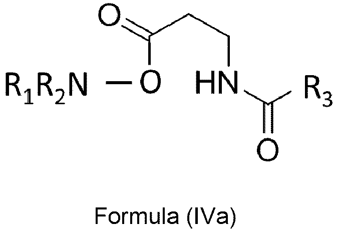
wherein R1 and R2, together with the N atom to which they are bound, form a heterocyclic group with 4 to 7 carbon atoms, optionally substituted with one or more oxo groups; and
wherein R₃ corresponds to an alkyl group having 1 to 6 carbon atoms (C₁-C₆),
or any pharmaceutically acceptable salts, esters, tautomers, solvates, and hydrates thereof, or any combinations thereof;
and
d) Clav 7, of formula (V): 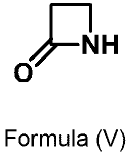 or any pharmaceutically acceptable salts, esters, tautomers, solvates, and hydrates thereof, or any combinations thereof.

2. The compounds according to claim 1, wherein Clav 2 is bis-((isobutoxycarbonyl)oxy)-1,3-dioxopropanoic anhydride, with formula (VI): 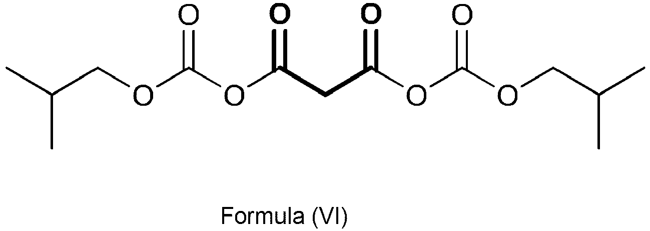 or any pharmaceutically acceptable salts, esters, tautomers, solvates, and hydrates thereof, or any combinations thereof.

3. The compounds according to claim 1, wherein Clav 5 is 3,3-diethoxypropanoic anhydride, with formula (VII): 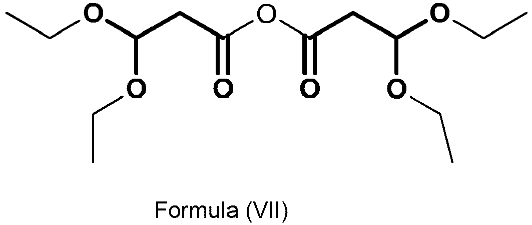 or any pharmaceutically acceptable salts, esters, tautomers, solvates, and hydrates thereof, or any combinations thereof.

4. The compounds according to claim 1, wherein Clav 6 is 2,5-dioxopyrrolidin-1-yl 3-acetamidopropanoate anhydride, with formula (VIII): 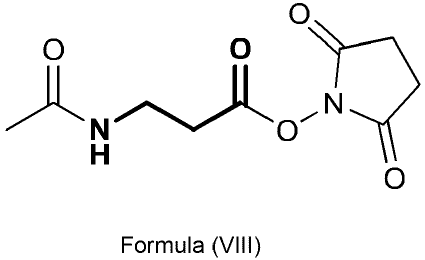 or any pharmaceutically acceptable salts, esters, tautomers, solvates, and hydrates thereof, or any combinations thereof.

5. A composition comprising or consisting of at least one compound according to any of claims 1 to 4, or any combinations thereof.

6. The composition according to the preceding claim, wherein the concentration of Clav 2, Clav 5, Clav 6, and/or Clav 7 is between 4-8 mM.

7. The composition according to any of claims 5 to 6, wherein the concentration of Clav 2, Clav 5, Clav 6, and/or Clav 7 is 6 mM.

8. The composition according to any of claims 5 to 7, further comprising at least another active ingredient.

9. The composition according to any of claims 5 to 8, further comprising at least one pharmaceutically acceptable excipient.

10. The composition according to any of claims 5 to 9, further comprising at least one pharmaceutically acceptable vehicle.

11. A dosage form comprising at least one compound according to any of claims 1 to 4 or the composition according to any of claims 5 to 10.

12. The dosage form according to the preceding claim which is selected from the list consisting of: plaster, ointment, paste, cream, solution, suspension, emulsion, lotion, liniment, gel, hydrogel, hydrocolloid, foam, powder, or any combinations thereof.

13. The dosage form according to any of claims 11 to 12 which is a solution or suspension.

14. A kit or device comprising or consisting of at least one compound according to any of claims 1 to 4, the composition according to any of claims 5 to 10, or the dosage form according to any of claims 11 to 13.

15. The kit or device according to the preceding claim, further comprising the elements necessary for performing a basophil activation test (BAT).

16. The kit or device according to any of the preceding claims, further comprising at least one fluorescent monoclonal antibody suitable for the selection of basophils and the determination of the activation thereof, at least one stimulation buffer, at least one red blood cell lysis solution, at least one wash solution and human anti-IgE and N-formyl-methionyl-leucyl-phenylalanine and/or at least one positive control for the test.

17. Use of at least one compound according to any of claims 1 to 4, of the composition according to any of claims 5 to 10, of the dosage form according to any of claims 11 to 13, or of the kit or device according to any of claims 14 to 16 in the production of a medicinal product.

18. Use of at least one compound according to any of claims 1 to 4, of the composition according to any of claims 5 to 10, of the dosage form according to any of claims 11 to 13, or of the kit or device according to any of claims 14 to 16 in the production of a medicinal product for the prevention, identification, screening, and/or diagnosis of an allergy to clavulanic acid.

19. Use according to the preceding claim, wherein the compound, the composition, or the kit or device are applied on an isolated biological sample.

20. Use according to the preceding claim, wherein the isolated biological sample is blood.

21. An *in vitro* method of obtaining data for use for the identification, screening and/or diagnosis of an allergy to clavulanic acid in an individual, which method comprises:
a) quantifying the percentage of activated basophils in an isolated biological sample of said individual at the baseline, and
a') quantifying the percentage of activated basophils in an isolated biological sample of said individual after adding to same at least one compound according to any of claims 1 to 4, the composition according to any of claims 5 to 10, the dosage form according to any of claims 11 to 13, or applying the kit or device according to any of claims 14 to 16.

22. The method according to the preceding claim, which method further comprises:
b) calculating a stimulation index.

23. The method according to any of claims 21 to 22, wherein the quantification of the percentage of activated basophils of step a) is performed using the expression level of at least one basophil cell surface marker.

24. The method according to the preceding claim, wherein the basophil cell surface activation markers are selected from the list consisting of CD203c, CD63, CD13, CD107a, CD164, CD80, CD86, CD40L, HLA-DR, CD123, CRTh2, Ph-Creb, Ph-STAT5, Ph-S6rp, Ph-elIF4E, and CREB.

25. The method according to any of claims 21 to 24, wherein the basophil cell surface activation marker is CD63.

26. The method according to any of claims 21 to 25, wherein the stimulation index is the percentage of activated basophils in the quantification of step a') with respect to the percentage of activated basophils at the baseline of step a).

27. The method according to any of claims 21 to 26, wherein the isolated biological sample of step a) is blood.

28. The method according to any of claims 21 to 27, wherein additionally, the blood is previously incubated with a stimulation buffer and/or is centrifuged.

29. The method according to any of claims 21 to 28, wherein the quantification of markers is performed by means of flow cytometry.

30. A method for the prevention, identification, screening, and/or diagnosis of an allergy to clavulanic acid, which method comprises steps (a)-(b) according to any of claims 21 to 29, which method further comprises:
c) assigning the individual of step a') to the group of individuals with an allergy to clavulanic acid when the stimulation index calculated in step b) is equal to or greater than 2, more preferably greater than 2.5, and even more preferably greater than 3.
